Europäisches Patentamt

(19) European Patent Office

Office européen des brevets

(11) Numéro de publication: **0 070 941**
**B1**

(12) **FASCICULE DE BREVET EUROPEEN**

(45) Date de publication du fascicule du brevet:
02.04.86

(21) Numéro de dépôt: **81401217.5**

(22) Date de dépôt: **29.07.81**

(51) Int. Cl.⁴: **G 08 B 17/10,** G 01 N 27/12

(54) Circuit contrôlé de détection d'incendie par semi conducteur sensible au gaz et à faible consommation.

(43) Date de publication de la demande:
**09.02.83 Bulletin 83/6**

(45) Mention de la délivrance du brevet:
**02.04.86 Bulletin 86/14**

(84) Etats contractants désignés:
**AT BE CH DE FR IT LI LU NL SE**

(56) Documents cités:
**DE - A - 2 845 458**
**US - A - 3 860 919**
**US - A - 3 879 717**
**US - A - 3 955 186**
**US - A - 4 007 456**
**US - A - 4 088 986**
**US - A - 4 150 370**

(73) Titulaire: **Nudelmont, Jean-Claude, 130 avenue Jean-Pierre Timbaud, F-92400 Courbevoie (FR)**

(72) Inventeur: **Nudelmont, Jean-Claude, 130 avenue Jean-Pierre Timbaud, F-92400 Courbevoie (FR)**

(74) Mandataire: **Loyer, Bertrand et al, Cabinet Pierre Loyer 18, rue de Mogador, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Description

L'invention a trait à un circuit contrôlé de détection d'incendie par semi-conducteur sensible au gaz et à faible consommation.

Les montages connus utilisant un détecteur d'incendie par semi-conducteur sensible au gaz, comme celui décrit dans le document US-A-4 007 456, présentent le double inconvénient de fonctionner avec une forte consommation de courant et d'être sensibles à des gaz non caractéristiques d'incendies.

La consommation élevée de courant, de l'ordre de 0,5 à 1 watt par élément détecteur, a pour conséquence l'obligation d'employer des câblages de caractéristiques bien définies et l'obligation de recourir à des batteries d'accumulateurs de secours de très forte capacité – accroissant ainsi l'encombrement du dispositif et son prix de revient.

La sensibilité à des gaz non caractéristiques d'un incendie risque d'entraîner le déclenchement d'alarme même en absence de monoxyde de carbone, ces détecteurs étant sensibles notamment à de nombreux hydrocarbures – et divers produits de nettoyage.

L'objet de la présente invention est un circuit contrôlé de détection d'incendie par semi-conducteur sensible au gaz et à faible consommation, comprenant d'une part un élément semi-conducteur de résistance électrique variant avec la nature et la quantité des gaz et fumées auxquels il est exposé, d'autre part un filament de chauffage dudit élément, caractérisé en ce que l'on connecte en série le semi-conducteur et son filament chauffant de sorte qu'ils sont parcourus par le même courant.

Ainsi, contrairement aux circuits détecteurs antérieurs où le filament chauffant était parcouru par un courant relativement important I dont la variation était non significative lors de la variation de la résistance de l'élément semi-conducteur en présence de gaz provenant d'incendies, l'expérience montre d'une part que l'on peut appliquer aux bornes du circuit de détection lors de sa mise en service une tension de l'ordre de 5 à 20 volts et obtenir un courant i stable parcourant l'élément semi-conducteur et le filament d'autre part, obtenir un accroissement non négligeable $\Delta i$ de ce courant au cours de la détection de la présence du monoxyde de carbone, cet accroissement, résultant de la diminution de résistance du semi-conducteur, facilitant le retour de la position d'équilibre initial après disparition du monoxyde de carbone.

Un autre objet de l'invention est de limiter en outre la consommation d'un tel circuit en cas de saturation de l'élément détecteur semi-conducteur lors d'un excès de monoxyde de carbone par la mise en série d'une résistance de valeur prédéterminée avec le circuit série élément détecteur semi-conducteur, filament.

L'expérience montre que l'on parvient à obtenir alors, pour des tensions de l'ordre de 5 à 20 volts, un point d'équilibre stable en dépit de variations extérieures de température de l'ordre de -20 à +80 par exemple tout en réduisant la consommation du circuit qui devient 50 fois plus faible que la consommation des éléments usuels.

En outre lorsqu'une telle condition est atteinte on constate que l'élément semi-conducteur est tout particulièrement sensible au monoxyde de carbone et proportionnellement beaucoup moins sensible aux autres gaz.

Un autre objet de l'invention est de maintenir la très faible consommation de courant de détection lors de la transmission du signal de détection de la présence du monoxyde de carbone par diminution de la résistance de l'élément semi-conducteur en série avec le filament par la combinaison d'un circuit de détection et d'un circuit de retard contenant un condensateur, le circuit de détection entraînant l'émission d'un signal de courte durée par décharge du condensateur exclusivement lors d'une détection du passage de la résistance de l'élément semi-conducteur, en absence de monoxyde de carbone, à un autre état.

Il en résulte une consommation de courant dérisoire, l'émission n'ayant lieu que lors de la détection de la présence de monoxyde de carbone.

Une autre caractéristique de l'invention est d'utiliser la faible tension nécessaire à la détection pour disposer en parallèle avec le circuit de détection le dispositif de contrôle de son fonctionnement au moyen d'une diode électroluminescente commandée par un circuit intermittent.

Ainsi, contrairement aux dispositifs antérieurs nécessitant une consommation de 20 à 30 mA pour transmettre l'information d'alarme résultant d'une diminution de la résistance de l'élément détecteur semi-conducteur, la transmission du signal de détection ne consomme que quelques microampères.

Une autre caractéristique de l'invention est de maintenir la consommation à un niveau extrêmement bas lors du traitement du signal de détection tout en assurant le contrôle d'un ensemble de circuits de détection de ce type, par la prévision d'un circuit commun du traitement des signaux caractérisé en ce que l'ensemble alimenté par la même tension comprend un circuit commun de transmission des signaux de détection connecté à un potentiel de référence commun et trois circuits de comparaison desdits signaux avec trois tensions de référence caractérisant l'alarme, le fonctionnement normal et le dérangement soit par coupure de la ligne de tension positive soit par coupure de la ligne de retour.

L'expérience montre que l'ensemble du système fonctionne parfaitement pour une consommation des circuits de détection de l'ordre de quelques milliampères, les circuits de traitement des signaux reçus ayant une consommation de l'ordre de 500 µA.

D'autres caractéristiques de la présente invention apparaîtront au cours de la description suivante faite en référence aux dessins annexés qui représentent à titre d'exemple non limitatif un mode de réalisation de la présente invention.

Sur les dessins:

La figure 1 est une représentation schématique des circuits de détection de l'élément détecteur à faible consommation électrique,

La figure 2 est la représentation schématique d'un ensemble de circuits de détection reliés à un même circuit de traitement des signaux et,

La figure 3 le schéma détaillé des circuits de traitement des signaux pour le contrôle général du fonctionnement normal ou défectueux de l'ensemble du système.

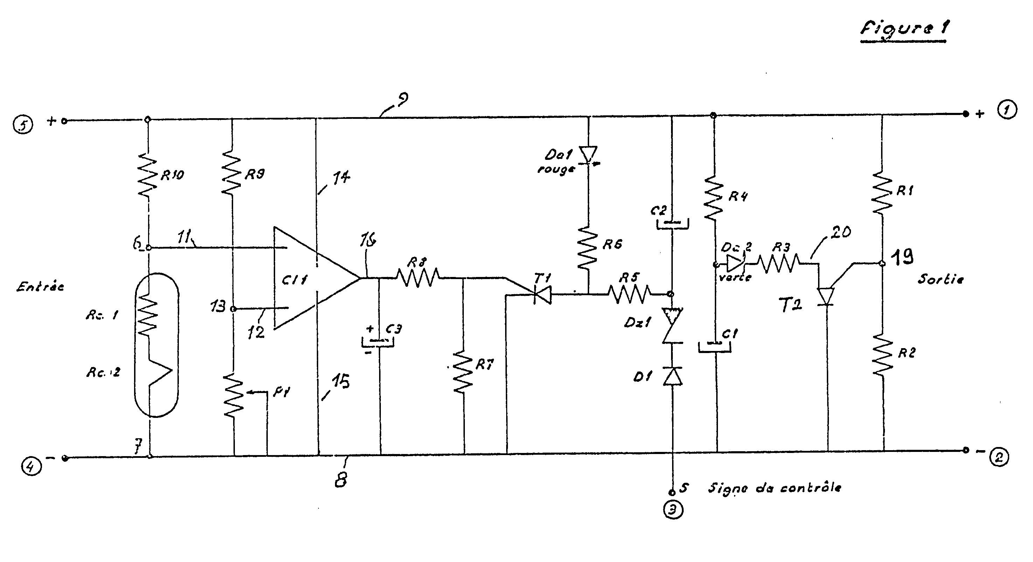

Le détecteur à faible consommation représenté figure 1 comprend un élément semi-conducteur détecteur de gaz constitué d'un élément semi-conducteur sensible représenté sous la forme de la résistance $Rc_1$, la valeur de cette résistance variant en fonction des gaz et fumées en contact avec l'élément semi-conducteur et un filament $Rc_2$ connecté en série avec le semi-conducteur de résistance variable $Rc_1$.

Afin d'éviter la présence de courant d'intensité trop élevée en cas de saturation de l'élément semi-conducteur lors d'un trop grand apport de monoxyde de carbone, une résistance $R_{10}$ est connectée à la borne 6 de l'élément semi-conducteur représenté par $Rc_1$.

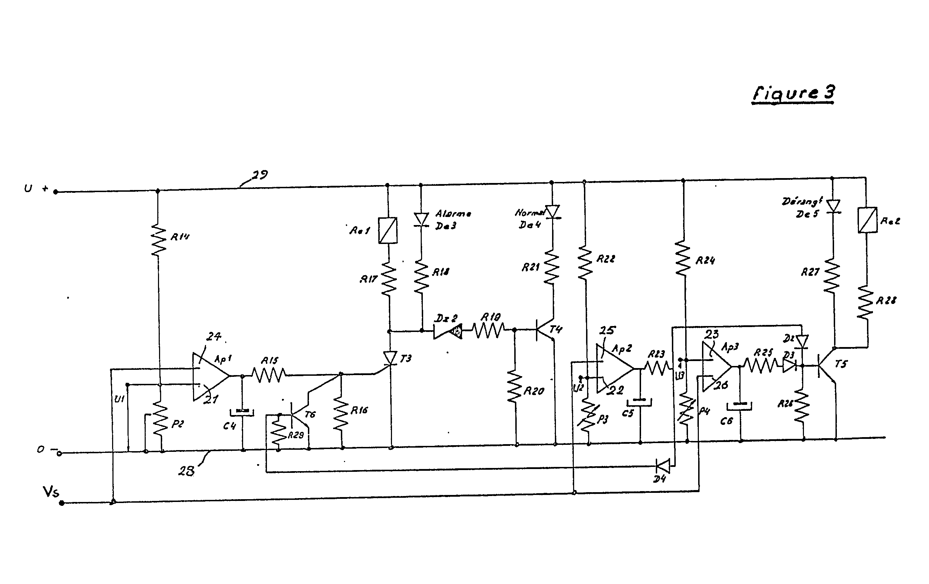

La borne 7 du filament $Rc_2$ est reliée à la ligne 8 recevant la tension d'entrée fournie par la ligne 28, figure 3, à la borne 2. La résistance $R_{10}$ est reliée à la ligne 9 connectée à la borne 1 recevant la tension d'alimentation de la ligne 29 figure 3 qui dans l'exemple représenté est de +12 v. La source de tension alimentant les lignes 28 et 29 pouvant être quelconque n'a pas été représentée.

Le signal détecté par l'élément détecteur $Rc_1$, $Rc_2$ est transmis par la borne 6 reliée par la connexion d'entrée 11 à l'amplificateur opérationnel $C_{11}$ dont la seconde connexion d'entrée 12 est connectée à la borne commune 13 de la résistance $R_9$ et du potentiomètre $P_1$. L'alimentation des circuits de l'amplificateur $C_{11}$ s'effectue par les connexions 14 et 15.

On règle le potentiomètre $P_1$ pour que la tension appliquée à l'amplificateur opérationnel $C_{11}$ par 12 soit inférieure à celle résultant de l'élément semi-conducteur de résistance $Rc_1$ de façon que le niveau de la sortie 16 de $C_{11}$ soit 0 lorsque cet élément n'est pas en contact avec le monoxyde de carbone.

Si V désigne la tension de la borne 6 et U celle de la borne 13 la sensibilité du système de détection dépend de la différence entre les valeurs V et U.

Dès que le monoxyde de carbone pénètre dans l'élément semi-conducteur de détection, la résistance $Rc_1$ de l'élément diminue de sorte que la valeur V diminue jusqu'à un seuil de déclenchement U où le potentiel V à la borne 6 devient inférieur ou égal au potentiel U de la borne 13.

La diminution de la résistance $Rc_1$ de l'élément semi-conducteur entraîne un léger accroissement de tension à la borne commune: filament $Rc_2$ – élément semi-conducteur $Rc_1$. L'accroissement du courant traversant le semi-conducteur et le filament $Rc_2$ se stabilisant, on constate que l'on conserve une consommation très faible tout en facilitant l'autonettoyage de l'élément détecteur lors de la dissipation du monoxyde de carbone et du retour de l'élément détecteur.

Dans le cas d'une saturation complète de l'élément semi-conducteur entraînant une grande diminution de la résistance $Rc_1$, la résistance $R_{10}$ limite le courant à une valeur prédéterminée qui peut être de l'ordre de 1 mA sur le détecteur.

Dès que le seuil de déclenchement est atteint c'est-à-dire lorsque la tension à l'entrée 11 de l'amplificateur opérationnel $C_{11}$ est inférieure ou égale à celle de l'entrée 12, la tension à la sortie 16 de l'amplificateur entraîne la conduction du thyristor $T_1$ ainsi que la diode électroluminescente d'alarme $De_1$ par le circuit ligne 9, $De_1$, $R_6$, $T_1$, ligne 8.

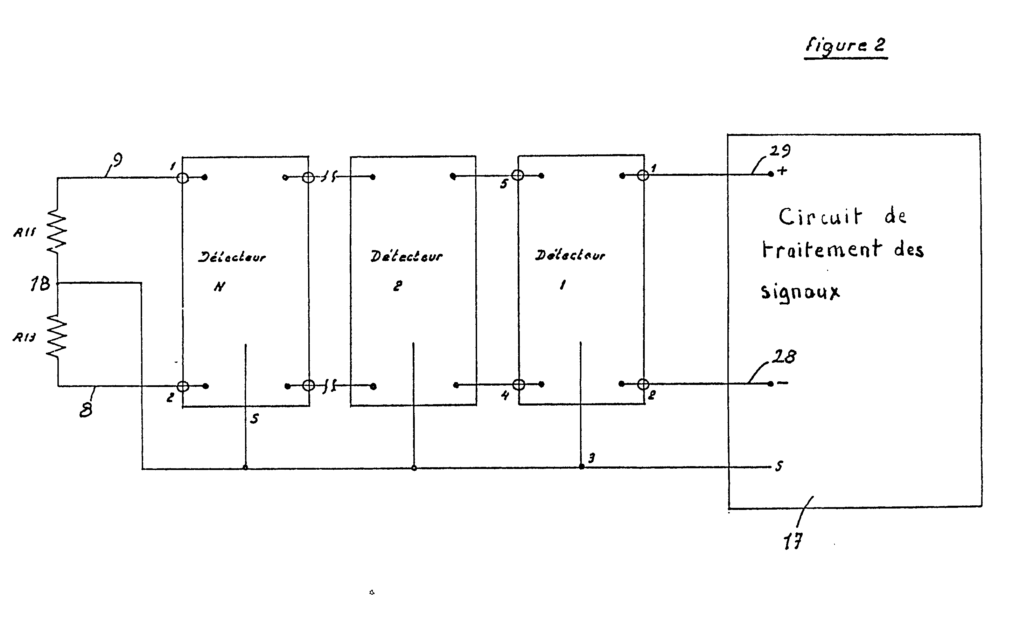

La transmission de ce déclenchement d'alarme s'effectue par un circuit particulier afin de rendre disponible ce signal très faible au circuit de traitement des signaux représenté figure 3. A cet effet, la transmission est retardée par le circuit de retard composé du condensateur $C_2$ et de la résistance $R_5$ permettant la transmission d'un signal d'alarme disponible à la borne 3 avec un retard de l'ordre de 1 ms. Ce signal est transmis par la diode $D_1$, la diode de Zener $DZ_1$, la borne 3 et le fil d'entrée S d'accès au circuit de traitement des signaux représenté schématiquement en 17 figure 2.

Sur cette figure on a représenté un ensemble de n détecteurs dont les bornes, portant les mêmes références sont raccordées pour établir la continuité de la ligne 8 de tension positive de l'ordre de 12 V par exemple et 9 de la tension 0 dans l'exemple indiqué.

La ligne de transmission S des signaux est connectée à la borne commune 18 des résistances $R_{11}$ et $R_{13}$ de façon à fournir une valeur de référence Vs.

Le contrôle du fonctionnement du détecteur est assuré par un circuit impulsionnel commandant l'allumage par top de la diode électroluminescente $De_2$ signalant le fonctionnement du détecteur. Les tensions appliquées au thyristor $T_2$ sont d'une part la tension de référence de la borne commune 19 des résistances $R_1$ et $R_2$, d'autre part la tension de l'entrée 20 connectée à la résistance $R_3$ en série avec la diode $De_2$. Lorsque le condensateur $C_1$ se charge à travers la résistance $R_4$ sa tension croît jusqu'à la valeur de la tension de référence de la borne 19. Lorsque cette valeur est atteinte, le thyristor $T_2$ devient conducteur entraînant une décharge rapide de $C_1$ à travers la diode $De_2$ ce qui a pour effet de diminuer la tension d'entrée 20 et de bloquer à nouveau le thyristor $T_2$.

Le contrôle de l'alimentation des circuits de commande des éléments semi-conducteurs de détection étant ainsi assuré par un moyen à très faible consommation de l'ordre de 500 μA on assure le contrôle du fonctionnement normal, du fonctionnement d'alarme, d'une coupure de la ligne de distribution 9 et de la ligne de distribution 8 au moyen de trois tensions de référence $U_1$, $U_2$ et $U_3$ que l'on détermine respectivement par le réglage des potentiomètres $P_2$, $P_3$ et $P_4$ figure 3. Ces tensions sont appliquées respectivement aux bornes 21, 22 et 23 des amplificateurs $Ap_1$, $Ap_2$ et $Ap_3$.

Les tensions de référence sont choisies pour que dans les conditions normales de fonctionnement leurs valeurs satisfassent aux inégalités suivantes $U > U_3 > U_1 > U_2 > 0$ (1) et $U_3 > Vs > U_2$ (2)

La ligne S de transmission des signaux étant connectée respectivement aux bornes 24, 25 et 26 des amplificateurs $Ap_1$, $Ap_2$ et $Ap_3$, les inégalités (2) entraînent une tension 0 à leurs bornes de sortie connectées respectivement aux condensateurs $C_4$, $C_5$ et $C_6$. Ces condensateurs ayant leur autre borne connectée à la ligne 28 de tension 0, les amplificateurs ne modifient pas la conduction des circuits, de sorte que le thyristor $T_3$ n'étant pas conducteur la diode de Zener $DZ_2$ est passante permettant la conduction du transistor $T_4$ et l'allumage normal de la diode électroluminescente $De_4$. L'allumage de cette diode est caractéristique du bon fonctionnement des éléments semiconducteurs de détection en absence de monoxyde de carbone.

Bien que la diode de Zener $DZ_2$ soit passante, la diode électroluminescente $De_3$ ne peut s'allumer en raison de la forte valeur de la résistance $R_{19}$.

Dès qu'un signal d'alarme apparaît, la ligne S transmet un signal retardé et la valeur de la tension Vs est telle que $U_1 > Vs > U_2$. Il en résulte que les conditions d'entrées appliquées à l'amplificateur $Ap_1$ sont modifiées et que sa tension de sortie rend conducteur le thyristor $T_3$ entraînant l'allumage de la diode électroluminescente d'alarme $De_3$ ainsi que l'excitation du relais d'alarme $Re_1$ se trouvant chacun dans un des circuits en parallèle contenant les résistances $R_{17}$ ou $R_{18}$. La chute de tension en résultant à la borne commune $R_{17}$–$R_{18}$ bloque la diode de Zener $DZ_2$ de sorte que le transistor $T_4$, devenant non conducteur, éteint la diode $De_4$ indicatrice du fonctionnement normal.

En cas d'une coupure d'une ligne, on détecte d'une part la coupure entraînant un manque de tension sur la ligne 9, d'autre part la coupure entraînant le manque de tension sur la ligne 8.

Si le fil de liaison 9 est coupé, la tension de référence Vs devient celle de la ligne 8 soit 0. Dans ce cas les sorties des amplificateurs $Ap_1$ et $Ap_2$ entraînent les conductions de $T_3$ et du transistor $T_6$. La conduction de $T_6$ entraîne alors celle du transistor $T_5$, laquelle entraîne l'allumage de la diode électroluminescente $De_5$, en série avec la résistance 27, et du relais indicateur de dérangement $Re_2$, en série avec la résistance $R_{28}$.

Si le fil de liaison 8 est coupé, la tension du fil S est celle de la ligne 9, soit 12 volt dans l'exemple considéré de sorte que seul l'amplificateur $Ap_3$ fournit une tension de sortie non nulle. Dans ces conditions le transistor $T_5$ est rendu conducteur, entraînant comme précédemment l'allumage de la diode électroluminescente $De_5$ et l'excitation du relais $Re_2$.

## Revendications

1. Circuit contrôlé de détection d'incendie par semi-conducteur sensible au gaz et à faible consommation, comprenant d'une part un élément semi-conducteur ($Rc_1$) de résistance électrique variant avec la nature et la quantité de gaz et fumées auxquels il est exposé, d'autre part un filament de chauffage ($Rc_2$) dudit élément, caractérisé en ce que l'on connecte en série le semi-conducteur et son filament chauffant de sorte qu'ils sont parcourus par le même courant.

2. Circuit tel que revendiqué en 1 dont le courant unique traversant le filament de chauffage ($Rc_2$) disposé en série avec l'élément semi-conducteur ($Rc_1$) est limité par une résistance ($R_{10}$) de valeur prédéterminée disposée dans le même circuit série.

3. Circuit tel que revendiqué dans l'une quelconque des revendications 1 et 2 dont la tension variable du semi-conducteur est appliquée à un amplificateur opérationnel ($C_{11}$), caractérisé en ce que l'émission du signal de détection du monoxyde de carbone s'effectue par la décharge d'un condensateur ($C_3$) sous le contrôle de l'amplificateur.

4. Circuit tel que revendiqué en 3 comprenant un circuit de déclenchement d'alarme relais ($Re_1$), une diode électroluminescente ($De_3$) et un circuit du témoin ($De_4$) de fonctionnement normal sans alarme intégrés à un circuit général de traitement des signaux (17), ce dernier incluant au moins un premier circuit de comparaison et d'amplification ($Ap_1$) de la tension (Vs) du signal transmis lors de la détection d'une diminution de la résistance ($Rc_1$) de l'élément détecteur de gaz et d'une tension prédéterminée ($U_1$) obtenue à partir des lignes (29–28) d'alimentation en tension, le dispositif de comparaison maintenant l'alimentation du circuit témoin du fonctionnement normal du détecteur de gaz lorsque la valeur de la tension (Vs) de la ligne de transmission est supérieure à la tension prédéterminée ($U_1$) et coupant ce circuit pour commander le circuit d'excitation de déclenchement d'alarme ($Re_1$) lorsque le signal (Vs) a une tension inférieure à ($U_1$).

5. Circuit tel que revendiqué en 4 comportant en outre un second circuit de comparaison et d'amplification ($Ap_2$) recevant le signal (Vs) du circuit détecteur de gaz et une tension prédéterminée ($U_2$), caractérisé en ce que ledit amplificateur déclenche un signal de dérangement du système lorsque la tension (Vs) est inférieure à la tension ($U_2$) caractéristique de la coupure de la ligne d'alimentation (9) en tension du circuit détecteur.

6. Circuit tel que revendiqué en 3 comportant en outre un troisième circuit de comparaison et d'amplification ($Ap_3$) recevant le signal (Vs) du circuit détecteur de gaz et une tension prédéterminée ($U_3$), de valeur telle que ledit circuit déclenche un signal témoin de dérangement ($Re_2$) lorsqu'il détecte une tension (Vs) supérieure ou égale à la tension ($U_3$); caractéristique de la cou-

pure de la seconde ligne d'alimentation (8) du circuit détecteur.

7. Circuit tel que revendiqué dans l'une quelconque des revendications 3 à 6 comprenant un ensemble de circuits détecteurs de gaz, caractérisé en ce que la ligne commune (Vs) de transmission des signaux détectés est connectée à un point commun (18) de résistances (R$_{11}$–R$_{13}$) d'un circuit monté en parallèle avec les détecteurs alimentés par des lignes d'alimentation communes (8, 9).

8. Circuit tel que revendiqué dans l'une quelconque des revendications 1 à 7, caractérisé en ce que chaque détecteur comprend en outre un circuit intermittent d'allumage (R$_4$, C$_1$, T$_2$) d'une diode électroluminescente (De$_2$) témoin de l'alimentation en tension de l'élément détecteur.

## Patentansprüche

1. Gasempfindlicher halbleitergesteuerter Brandmelderschaltkreis mit geringem Verbrauch, der einerseits ein Halbleiterelement (Rc$_1$) mit einem mit Art und Menge der Gase und des Rauches, denen er ausgesetzt ist, variierenden elektrischen Widerstand und andererseits einen Heizdraht (Rc$_2$) für das genannte Element aufweist, dadurch gekennzeichnet, dass der Halbleiter und sein Heizdraht in Reihe geschaltet werden, so dass sie durch den gleichen Strom durchströmt werden.

2. Schaltkreis nach Anspruch 1, dadurch gekennzeichnet, dass der durch den mit dem Halbleiterelement (Rc$_1$) in Reihe geschalteten Heizdraht (Rc$_2$) fliessende Strom durch einen in dem gleichen Reihenschaltkreis angeordneten Widerstand (R$_{10}$) mit bestimmtem Wert begrenzt wird.

3. Schaltkreis nach den Ansprüchen 1 bis 2, bei dem die variable Spannung des Halbleiters an einen Betriebsverstärker (C$_{11}$) angelegt wird, dadurch gekennzeichnet, dass die Abgabe des Detektorsignals für Kohlenmonoxyd durch die durch die Steuerung des Verstärkers erfolgende Entladung eines Kondensators (C$_3$) erfolgt.

4. Schaltkreis nach Anspruch 3, gekennzeichnet durch einen Relais-Alarmauslöser-Schaltkreis (Re$_1$), eine elektro-lumineszente Diode (De$_3$) und einen Vergleicherschaltkreis (De$_4$) für Normalbetrieb ohne Alarm, die in einem allgemeinen Signalaufbereitungs-Schaltkreis (17) integriert sind, wobei letzterer mindestens einen ersten Vergleicher- und Verstärkerschaltkreis (Ap$_1$) für die Spannung (Vs) des Signals bei Erfassung einer Verringerung des Widerstandes (Rc$_1$) des Gasdetektorelementes und eine vorherbestimmte Spannung (U$_1$) aufweist, die aus den Versorgungsspannungsleitungen (29–28) erhalten wird, wobei die Vergleichervorrichtung die Versorgung des Vergleicherschaltkreises für den normalen Betrieb des Gasdetektors aufrechterhält, wenn der Wert der Spannung (Vs) der Übertragungsleitung grösser ist als die vorherbestimmte Spannung (U$_1$), und wobei dieser Schaltkreis unterbrochen wird, um den Erregerschaltkreis für das Auslösen des Alarms (Re$_1$) anzusteuern, wenn

das Signal (Vs) eine Spannung unter (U$_1$) hat.

5. Schaltkreis nach Anspruch 4, der ausserdem einen zweiten Vergleicher- und Verstärkerschaltkreis (Ap$_2$) aufweist, der ein Signal (Vs) des Gasdetektorschaltkreises und eine vorherbestimmte Spannung (U$_2$) erhält, dadurch gekennzeichnet, dass der genannte Verstärker dann ein Systemstörungssignal abgibt, wenn die Spannung (Vs) geringer ist als die Spannung (U$_2$), welche für das Unterbrechen der Versorgungsspannungsleitung (9) des Detektorschaltkreises massgebend ist.

6. Schaltkreis nach Anspruch 3, der ausserdem einen dritten Vergleicher- und Verstärkerschaltkreis (Ap$_3$) aufweist, der das Signal (Vs) des Gasdetektorschaltkreises und eine vorherbestimmte Spannung (U$_3$) mit einem solchen Wert erhält, dass der genannte Schaltkreis ein Vergleichsstörsignal (Re$_2$) auslöst, wenn er eine Spannung (Vs) grösser oder gleich der Spannung (U$_3$) erfasst, die für die Unterbrechung der zweiten Versorgungsspannungsleitung (8) des Detektorschaltkreises massgebend ist.

7. Schaltkreis nach den Ansprüchen 3 bis 6 mit einer Einheit von Gasdetektorschaltkreisen, dadurch gekennzeichnet, dass die gemeinsame Leitung (Vs) für die Übermittlung der erfassten Signale mit einem gemeinsamen Punkt von Widerständen (R$_{11}$–R$_{13}$) eines zu den von den gemeinsamen Versorgungsspannungsleitungen (8, 9) mit Strom versorgten Detektoren parallelen Schaltkreises verbunden ist.

8. Schaltkreis nach den Ansprüchen 1 bis 7, dadurch gekennzeichnet, dass jeder Detektor ausserdem einen intermittierenden Zündschaltkreis (R$_4$, C$_1$, T$_2$) und eine elektro-lumineszente Diode (De$_2$) zur Prüfung der Spannungsversorgung des Detektorelementes aufweist.

## Claims

1. A monitored circuit for detecting fire by a gas-sensitive semiconductor, with a low level of consumption, comprising on the one hand a semiconductor element (Rc$_1$) with an electrical resistance varying with the nature and the amount of gas and fumes to which it is exposed, and on the other hand a heating filament (Rc$_2$) for said element, characterised in that the semiconductor and its heating filament are connected in series whereby the same current passes therethrough.

2. A circuit as claimed in claim 1 wherein the single current passing through the heating filament (Rc$_2$) disposed in series with the semiconductor element (Rc$_1$) is limited by a resistor (R$_{10}$) of predetermined value disposed in the same series circuit.

3. A circuit as claimed in either one of claims 1 and 2 wherein the variable voltage of the semiconductor is applied to an operational amplifier (C$_{11}$) characterised in that emission of the carbon monoxide detection signal is effected by the discharge of a capacitor (C$_3$) under the control of the amplifier.

4. A circuit as claimed in claim 3 comprising a circuit for triggering an alarm relay (Re₁), a light emitting diode (De₃) and a signalling circuit (De₄) for signalling normal operation without alarm, which are integrated in a general signal processing circuit (17), the latter including at least one first circuit for comparison and amplification (Ap₁) of the voltage (Vs) of the signal transmitted upon detection of a reduction in the resistance (Rc₁) of the gas detector element and a predetermined voltage (U₁) obtained from the voltage supply lines (29–28), the comparison device maintaining the feed for the circuit for signalling normal operation of the gas detector when the value of the voltage (Vs) of the transmission line is higher than the predetermined voltage (U₁) and cutting said circuit to actuate the circuit for exciting triggering of the alarm (Re₁) when the signal (Vs) is of a voltage of less than (U₁).

5. A circuit as claimed in claim 4 further comprising a second comparison and amplification circuit (Ap₂) for receiving the signal (Vs) from the gas detector circuit and a predetermined voltage (U₂), characterised in that said amplifier triggers a system fault signal when the voltage (Vs) is lower than the voltage (U₂) characteristic of a break in the voltage supply line (9) of the detector circuit.

6. A circuit as claimed in claim 3 further comprising a third comparison and amplification circuit (Ap₃) for receiving the signal (Vs) from the gas detector circuit and a predetermined voltage (U₃) of a value such that said circuit triggers a fault signalling signal (Re₂) when it detects a voltage (Vs) which is higher than or equal to the voltage (U₃) characteristic of a break in the second supply line (8) of the detector circuit.

7. A circuit as claimed in any one of claims 3 to 6 comprising an assembly of gas detector circuits characterised in that the common line (Vs) for transmission of the detected signals is connected to a common point (18) of resistors (R₁₁–R₁₃) of a circuit disposed in parallel with the detectors supplied by common supply lines (8, 9).

8. A circuit as claimed in any one of claims 1 to 7 characterised in that each detector further comprises an intermittent lighting circuit (R₄, C₁, T₂) for a light emitting diode (De₂) for signalling the supply of voltage to the detector element.

**0 070 941**

Figure 1

figure 2

Circuit de traitement des signaux

Détecteur N

Détecteur 2

Détecteur 1

R11

R13

0070941

*figure 3*